Europäisches Patentamt

**European Patent Office**

**Office européen des brevets**

(19)

(11) Publication number: **0 241 400**
A2

(12)

# EUROPEAN PATENT APPLICATION

(21) Application number: **87500009.3**

(22) Date of filing: **16.03.87**

(51) Int. Cl.³: **C 07 C 53/122**
**C 07 C 51/41, A 23 B 9/00**

(30) Priority: **20.03.86 ES 553196**

(43) Date of publication of application:
**14.10.87 Bulletin 87/42**

(84) Designated Contracting States:
**AT CH DE FR GB IT LI**

(71) Applicant: **ANAGALIDE, S.A.**
**Carretera, num. 5**
**E-22313 Pozan de Vero (Huesca)(ES)**

(72) Inventor: **Cavero Buera, José-Cruz**
**Maladeta, 20**
**22300 Barbastro (Huesca)(ES)**

(54) Process for the production of ammonium dipropionate and its use in food preservatives.

(57) Process for the production of Propionic Acid, ammonium salt (2:1) and derived products, useful in the preservation of foods for human and animal consumption.

The production of Propionic Acid, ammonium salt (2:1) is realized by means of a reaction of one mole of Ammonium Bicarbonate for every two moles of Pripionic Acid, without : use of solvent.

Propionic Acid, ammonium salt (2:1) obtained this way, is formulated as a liquid or solid (on a carrier of high surface area).

EP 0 241 400 A2

Croydon Printing Company Ltd.

## PROCESS FOR THE PRODUCTION OF PROPIONIC ACID, AMMONIUM SALT (2:1) AND DERIVED PRODUCTS, USEFUL IN THE PRESERVATION OF FOODS FOR HUMAN AND ANIMAL CONSUMPTION.

The present invention refers to a process for the production of Propionic Acid, ammonium salt (2:1) and derived products, useful in the preservation of foods (human and animal).

The growing need to increase the agricultural and animal production, together with every day greater demand of industrialization and rationalization of the agricultural and farming sector, have created a problem of storage, regulation and distribution of the raw materials, for human and animal consumption (mainly cereals) and also of the poultry and livestock feed, which has enlivened an always preoccupant theme: the preservation of foods.

Practically all natural organic materials suffer variations during storage.

One of the most important factors of these changes is the microbiological phenomenon.

Vegetable matters carry in themselves a microbial load (molds, yeast and bacteria) which, in certain circumstances, find in them a nutritive substratum and possibilities for their development.

Among the factors which exert an influence, the following are outstanding: humidity, temperature, the pH, the redox potential, the partial oxygen pressure, the same components of the nutritive substratum, etc.

If the microbial growth is important, it carries with it a series of physical changes (compactness, abnormal smell, loss of weight,...), nutritive changes (decomposition of carbohydrates, denaturalization of proteins, lipolysis of fats, degradation of vitamins,...), and the risk of production (due to some fungi) of mycotoxins, proliferation of bacteria which may cause intoxications or nutritional toxiinfections, being vehicles of pathogenic microorganisms that can cause serious infections.

It's obvious then the significance of eliminating this potential risk which is caused by a poor preservation of foods for human and animal nutrition.

For this, it is necessary to dispose of preservatives that are active, innocuous, and under priced.

Propionic Acid is an active matter that unites these three qualities.

Already introduced in 1938 was the utilization of Propionic Acid and propionates like preservatives in the bread and pastry industry (US Patent 2.154.449).

Also in 1938, PECK, S.M., and ROSENFELD, J., discovered the antifungal action of Propionic Acid at concentrations of 0.004 moles/liter.

Since then a great quantity of studies, patents and applications of Propionic Acid and of the propionates in the preservation of foodstuffs (human and animal) were published.

However since Propionic Acid is liquid, corrosive and inflammable, the difficulties of manipulation have made that habitually propionates are preffered, that, even though they present a minor preservative action, they have qualities of superior handling.

In the latter years appeared products in which the Propionic Acid is absorbed in different carriers (US Patent 3.812.269, US Patent 4.199.606), that even though permitting its handling as free-flowing powder, less inflammable and corrosive, having an abundant release of acidic vapours, that although contribute to the preservative action by a gas-solid contact, is difficult and troublesome its manipulation and provides effectiveness only for a short time.

The mayor part of the propionates, used as preservatives, are solid products in which the Propionic Acid is salified with sodium, potassium, calcium,... In these compounds the chemical element which salifies doesn't give an antimicrobial action by itself, furthermore contributing so that the pH of the environment to which its applied nears neutrality (with the consequent loss of antimicrobial activity).

In Ammonium Propionate (extremely hygroscopic product, which is obtained and handled ordinarily as liquid), the ammonium has its own antimicrobial action, basically antifungal (BOTHAST, R.J., et al., J. of Dairy Science, 56 (1973) 241-245), but remains being a product of neutral pH.

Propionic Acid, ammonium salt (2:1), also called Ammonium Acid Propionate, Ammonium Dipropionate or Propionic Acid half-neutralised with Ammonia, is a product that being partially alone salified with Ammonia, keeps a pH acid (pH 1:100 ≅ 4.8).

The pH acid is the most favorable for the antimicrobial action of Propionic Acid and its derivatives, but furthermore according to the studies of WOOLFORD, M.K., (Grass and Forage Science, 39 (1) (1984) 75-79) at pH 5 the minimum inhibitory concentration (mmole/liter) of Propionic Acid, ammonium salt (2:1) against bacteria and fungi, is inferior to the Ammonium Propionate and even to the Propionic Acid.

The known method of the production of Propionic Acid, ammonium salt (2:1) is based on the reaction of Propionic Acid with Ammonia in an aqueous solution (British Patent 1.505.388).

Since the maximum concentration of ammonia in aqueous solutions, should be 34% weight/weight (d= 0.88), obtained are solutions of Propionic Acid, ammonium salt (2:1) with approximately 17% water. This amount of water greatly hinders its transformation in powdered products with higher contents in active substance, dry and free-flowing.

But over all, the principal object of the present invention is to provide a new process for the production of Propionic Acid, ammonium salt (2:1), (also called Ammonium Acid Propionate, Ammonium Dipropionate, etc.) which permits its preparation in concentrated form, in great quantities and at low price.

Another object of this invention is to prepare from Propionic Acid, ammonium salt (2:1), obtained in this way, products in powder or liquids which allow its easy and practical usage as preservatives in (human and animal) nutrition.

The new procedure for the production of Propionic Acid, ammonium salt (2:1), consists in making a direct reaction and without any solvent, in a container or adecuate stainless steel reactor, one mole of Ammonium Bicarbonate for every two moles of Propionic Acid.

It produces an endothermic reaction, with release of carbonic anhydride which helps the displacement of the equilibrium obtaining a practically total yield.

That which the process is realized without other presence of solvent that the water generated in the same reaction, helps to obtain a concentrated product (approximately 89% Propionic Acid, ammonium salt (2:1) + approximately 11% water).

It's convenient, by means of heating at 30-35°C, to avoid a high fall of the temperature in the reactioning mixture because it would decrease the rate of the reaction and even can freeze the the whole reactioning mass.

Do to the use of Propionic Acid, ammonium salt (2:1), it's important that both Propionic Acid and Ammonium Bicarbonate should be of food grade.

In view of the fact that Propionic Acid, ammonium salt (2:1) 89%, tends to deposit crystals approximately under 20°C, its convenient for its practical use to formulate adequately (in powder or liquid) according its concrete application as preservative.

Applicating it to cereals or products with destination to human nutrition, it's preffered not to add inactive watter, dilute simply with purified water obtaining concentrations of 80% Propionic Acid, ammonium salt (2:1), and inferiors.

To apply it to raw materials for animal feed or compound feeds, it can become absorbed in carriers of low density and high capacity of absorbtion like expanded vermiculite or better in precipitated or pyrogenic silicas of high surface area (greater than 100 m$^2$/g according to BET).

In this way is obtained a dry and free-flowing powder with concentrations of up to 60% Propionic Acid, ammonium salt (2:1) and inferiors.

In systems of automatic addition to raw materials for animal nutrition or compound feeds it can be convenient to use a product in liquid form. In these cases purified water is added to the Propionic Acid, ammonium salt (2:1) approx. 89% to dilute it, and Propylene Glycol can also be added (5-15% w/w of the total formulation) to prevent freezing at low temperatures. In this way liquid formulations can be obtained with a content of 75% Propionic Acid, ammonium salt (2:1) and inferiors.

These liquid formulations can also be applied to drinking water of animals for the control of fungi and the prevention of coli-bacillary enteritis.

Next are given a few examples in order to illustrate the present invention.

Example 1

Process for the production of Propionic Acid, ammonium salt (2:1).

A stainless steel reactor of 2000 l capacity, supplied with a heating device, discharge valve, stirring mechanism and a evacuation tube of gases, is loaded with 560 kg of Ammonium Bicarbonate 94-96% and then is slowly added 1010 kg of Propionic Acid 98-99% Immediately it produces an endothermic reaction with release of carbonic anhydride. To avoid a high fall of the temperature of the reactioning mass, its convenient that, once approximately the half of the Propionic Acid is added, starting the heating between 30-35°C.

When the releasing of carbonic anhydride ceases, its stirred for some minutes and the reaction is finalized; approximately 1250 kg of a transparent or weakly yellowish liquid is obtained, somewhat viscous with pungent odour, containing approximately 89% Propionic Acid, ammonium salt (2:1), (approximately equal to 80% Propionic Acid).

Example 2

Process for the production of a concentrated liquid product usable as preservative of cereals with utility for the human consumption.

A stainless steel container of 1200 l capacity, provided with a discharge valve and stirring device, is loaded with 880 kg of Propionic Acid, ammonium salt (2:1) 89%, and 120 kg of purified water is added. The stirring is put in motion during some minutes to homogenously mix the components; 1000 kg of a colourless or weakly yellowish solution is obtained, containing approximately 78% of Propionic Acid, ammonium salt (2:1), (approximately equivalent to 70% of Propionic Acid).

Example 3

Process for the production of a powdered product (with vermiculite) usable as a preservative of raw materials for animal nutrition or compound feeds.

A stainless steel mixer of 6000 l capacity, with a liquid adding device, is loaded with 560 kg of expanded vermiculite of 0.5-2 mm medium size. Immediately putting in motion the mixer, by the liquid adding device, are pumped 440 kg of Propionic Acid, ammonium salt (2:1) 89%. The mixing action is continued during 10-15 minutes. After this time, the mixer is stopped, obtaining 1000 kg of a powdered product, dry and free-flowing, containing approximately 39% Propionic Acid, ammonium salt (2:1), (approximately equivalent to 35% Propionic Acid).

Example 4

Process for the production of a powdered product (with precipitated silica) usable as a preservative of raw materials for animal nutrition or compound feeds.

A suitable stainless steel mixer of 1500 l capacity, with a liquid adding device, is loaded with 370 kg of precipitated silica (of high surface area $\cong$ 200 m$^2$/g according to BET). Immediately putting in motion the mixer, by the liquid adding device, are pumped 630 kg of Propionic Acid, ammonium salt (2:1) 89%. The mixing action is continued during 10-15 minutes. After this time, the mixer is stopped, obtaining 1000 kg of a powdered product, dry and free-flowing, containing approximately 56% of Propionic Acid, ammonium salt (2:1), (approximately equivalent to 50% Propionic Acid).

Example 5

Process for the production of a concentrated liquid product usable as a preservative of raw materials for animal nutrition and compound feeds.

An identical container described in example 2, is loaded with 818 kg of Propionic Acid, ammonium salt (2:1) 89%, 82 kg of purified water and 100 kg of Propylene Glycol food grade. The stirring is put in motion during some minutes to mix the components homogenously; 1000 kg of a colourless or weakly yellowish solution is obtained containing approximately 73% Propionic Acid, ammonium salt (2:1), (approximately equal to 65% Propionic Acid).

Example 6

Process for the production of a liquid product usable as a preservative and preventive for the drinking water for animals.

An identical container as described in example 2, is loaded with 630 kg of Propionic Acid, ammonium salt (2:1) 89%, 219 kg of purified water and 150 kg of Propylene Glycol. The stirring is put in motion and 1 kg of an aqueous colouring solution (1:20) w/v of a mixture of Tartrazine (E-102) and Patent Blue V (E-131) is added.

The stirring is continued some minutes more to mix the preparation homogenously and 1000 kg of a clear green coloured solution is obtained, containing approximately 56% Propionic Acid, ammonium salt (2:1), (approximately equal to 50% Propionic Acid).

0241400

- 1 -

CLAIMS:

1.- Process for the production of Propionic Acid, ammonium salt (2:1), and derived products useful in the preservation of foods for human and animal consumption, which is characterized essentially for the reaction of one mole of Ammonium Bicarbonate for every two moles of Propionic Acid, without use of any solvent, maintaining the reaction temperature above 20°C and preferably 30-35°C.

2.- Process for the production of Propionic Acid, ammonium salt (2:1), and derived products useful in the preservation of foods for human and animal consumption, according to the previous claim, wherein to the Propionic Acid, ammonium salt (2:1) resultant, purified water or a mixture of purified water and Propylene Glycol, is added for diluting its concentration and avoid crystallization at temperature below 20°C; the quantity of water in the final solution is equal or superior to 15%, while the quantity of Propylene Glycol in the same final solution is equal or superior to 5%.

3.- Process for the production of Propionic Acid, ammonium salt (2:1), and derived products useful in the preservation of foods for human and animal consumption, according to claims 1 and 2, wherein the Propionic Acid, ammonium salt (2:1) is added to a carrier of low density and high capacity of absorption or high surface, such as vermiculite or precipitated or pyrogenic silica of high surface area (greater than 100 m /g according to BET), obtaining a dry and free-flowing powder with a content in Propionic Acid, ammonium salt (2:1), up to 60%.